# EUROPEAN PATENT SPECIFICATION

(11) **EP 0 595 922 B1**
(45) Date of publication and mention of the grant of the patent: **05.02.1997**
(21) Application number: 92915859.0
(22) Date of filing: 23.07.1992
(51) Int. Cl.: G01N 33/02, G01N 33/14

(54) **A TEST METHOD**
Testverfahren
METHODE DE TEST

(30) Priority: 23.07.1991 GB 91158964
(43) Date of publication of application: 11.05.1994
(73) Proprietor: SOUS CHEF LIMITED, Deeside, Clwyd CH5 2NF (GB)
(72) Inventor: HAUSER, John Yniol, Sous Chef Ltd., Deeside, Clwyd CH5 2NF (GB)
(74) Representative: Pendlebury, Anthony
(86) International application number: GB9201357
(87) International publication number: WO9302357

(56) References cited:
- WO-A-87/07722

## Description

The present invention relates to a test method and, in particular, to a method of testing a procedure for processing a flowable material, such as a foodstuff or like organic material. The invention also relates to a method of determining an electrode or cell characteristic and, more particularly, it relates to a method of testing the performance of an ohmic heating apparatus.

In an ohmic heating process, an electrically conducting medium is heated by passing an alternating electric current between platinum/ iridium coated electrodes immersed in the material. The process was developed for heat treating flowable foodstuffs, prior to their being packed, and involves pumping a continuous flow of foodstuff through a heating chamber, containing the electrodes, where the passage of an electric current through the food raises its temperature sufficiently for sterilization to take place. An alternating electric current, rather than a direct current, is used in order to prevent electrolysis and consequential undesirable chemical modification of the heated foodstuff.

In principle, electrolysis of simple conducting aqueous solutions, will produce molecular products such as hydrogen, oxygen, chlorine, hydrogenperoxide etc., according to the exact nature of the conducting solute. These products are formed from reactive free radical intermediates generated at the electrodes' surfaces.

It was thought the use of an alternating current would prevent free radicals from being formed during the operation of an ohmic heating process. However, it has now been determined that, surprisingly, there remains a risk that chemical changes consistent with free radical activity, can be induced by an ohmic heating process, notwithstanding the use of an alternating electric current. If any such free radicals are generated, their presence could lead to unwanted and undesirable chemical modifications to organic material, of a type which would not be induced by normal "thermal" heating methods. Complex nutrients, such as proteins and vitamins, are considered to be particularly at risk. Since foodstuffs are highly complex and generally non-homogenous mixtures, which are, in any event, chemically and physically modified by conventional thermal heating (cooking) processes, it has not proven possible to determine directly whether ohmic heating processes cause chemical changes in food, which are not caused by conventional heating.

It is an aim of the present invention to provide a method of determining whether a procedure, such as ohmic heating, will cause undesirable chemical changes in a material subjected to the process.

According to the present invention, in a first aspect, there is provided a method for testing a procedure or apparatus for processing a flowable material, characterised by carrying out the procedure or operating the apparatus on a test fluid comprising a test substance which remains substantially unmodified under the intended influence of the apparatus or procedure and which, in the presence of a free radical, is modified into a detectably different form and, thereafter, analysing the test fluid for said detectably different form. Preferably, the flowable material is electrically conducting and may be a foodstuff or like organic material.

An advantage of the present invention is that adverse effects of changes to a procedure, including wear or damage to the apparatus employed for its operation, can be detected by carrying out the inventive test method after changes have been introduced, or on a regular basis. For example, the process parameters of an ohmic heating plant, such as the current, voltage, frequency and duration of exposure to current can be altered so as to minimize the amount of, or eliminate all of, said identifiably different species which is produced when the test method of the invention is carried out. In this way, the plant may be adjusted, or repaired, so that it will cause no more chemical changes in the material normally passing therethrough than would a conventional "thermal" heating process.

In an embodiment, the apparatus comprises an electrode and the method comprises contacting the electrode with the test fluid, feeding an alternating electric current to the electrode and, thereafter, analysing the test fluid for said detectably different form. Preferably the test fluid is electrically conducting and electric current is passed through the test fluid, to and from the electrode. More preferably, at least two electrodes are contacted with the fluid and the alternating current is passed between the electrodes.

An advantage of this latter embodiment of the invention is that, when carried out on a regular basis, deterioration of an electrode, or electrodes, which can often give rise to unwanted, free radical associated changes in a medium surrounding an electrode, can be detected through detecting the production of said detectably different forms or species. In procedures, such as ohmic heating processes, the only way in which the sort of damage which could give rise to free radical associated activity, could previously be detected was through removal and examination of the electrodes. Such a procedure is both time consuming and expensive and would be rendered substantially unnecessary by the practice of the present invention.

In a further embodiment, the apparatus comprises an electrolytic cell and the method comprises allowing the test fluid to enter the cell, passing an alternating electric current through the fluid and, thereafter, analysing the test fluid for said detectably different form.

Preferably, the procedure or apparatus is intended to heat the flowable material to an elevated temperature and, in the inventive test method, the procedure or operating the apparatus heats the test fluid to said elevated temperature, at which substantially all of the heated test substance will not be modified into said detectably different form by the heat alone. In a preferred embodiment, the procedure is electrical and is preferably an ohmic heating process, involving, in the test method, the passage of an alternating electric current through the test fluid, in order to raise its temperature to said elevated temperature.

In preferred embodiments of the invention, the test fluid and substance are substantially stable at temperatures of up to 120°C and preferably at temperatures of up to 140°C (at the pressure at which the test procedure is operated). Preferably the test substance is a heterocyclic base, amino acid or an essential fatty acid. Preferably the test substance is a pyrimidine or purine and is most preferably thymine. When the test substance is an essential fatty acid it is preferably linoleic acid. The test fluid is preferably an aqueous solution and, in addition to the aforementioned test substance, preferably includes sodium sulphate. The identifiably different species is preferably detected by HPLC techniques.

In a second aspect, the present invention provides a method of operating an ohmic heating plant, including the step of carrying out a method in accordance with the first aspect of the present invention, preferably, in any of its aforementioned preferred forms or embodiments.

The following examples are provided to illustrate the invention and are not intended to limit its scope in any way.

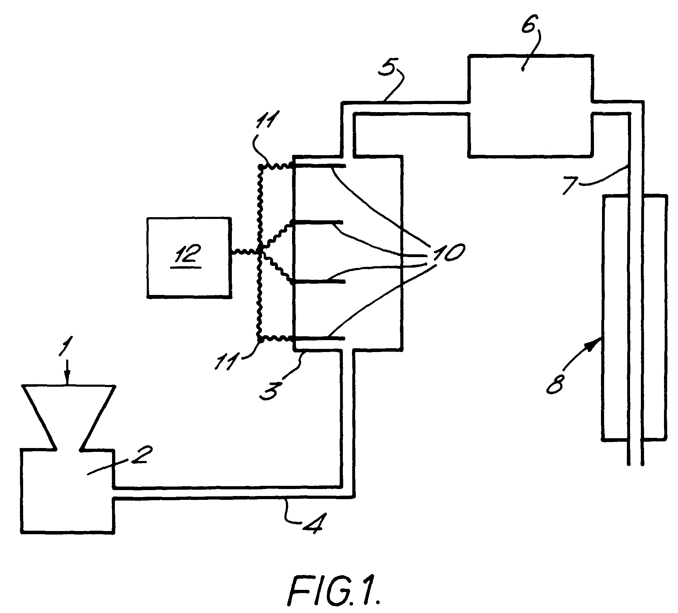

Figure 1 shows a schematic diagram of a typical ohmic heating plant.

A hopper 1 is provided at the inlet of a pump 2, which communicates with an ohmic heating cell 3, via a conduit 4. A second conduit 5 joins the ohmic heating column 3 to a holding tube 6 and a third conduit 7 extends from the holding tube 6, through a water cooling jacket 8, to an outlet 9.

Four electrodes 10, project into the ohmic heating column 3 and are joined, by cables 11 to an electrical supply 12, arranged to provide an alternating current to the electrodes 10.

In use, a flowable conducting medium, such as a foodstuff, is fed into the hopper 1 and the pump forces the foodstuff along the first conduit 4 and into the ohmic heating column 3. The ohmic heating column 3 is arranged so that the flowable medium moves from the first conduit 4, past all of the electrodes 10 and out via the second conduit 5. The flowable medium is hot on leaving the column 3 and is held at an elevated temperature in the holding tube 6. The medium, thereafter, passes from the holding tube 6 and out of the heating plant, via the third conduit 7, to be packed or further processed. Before leaving the ohmic heating plant, the medium is cooled down by the influence of the water cooling jacket 8 around the third conduit 7.

### Practice of the present invention using the apparatus of Figure 1.

An aqueous solution of 1 x 10⁻³ mol/dm³ of thymine was preheated to about 70°C and then passed through the apparatus shown in Figure 1. The solution was passed through the plant at two different rates, 430 and 740 litres per hour, and a 3-phase electric current (1500V, 6-10A, 50hz) was passed through the solution, between the electrodes 10. On leaving the ohmic heating column 3, the thymine solution passed at 740 litres per hour had been heated to 120°C and that passed at 470 litres per hour reached 140°C. The pressure within the ohmic heating cell was in the order of 4 bar. The thymine solutions passing out of the ohmic heating plant were analysed using HPLC measurements and no breakdown products were found. This procedure can be carried out using a 1 x 10⁻³ mol/dm³ aqueous solution of linoleic acid in place of thymine.

Thymine is a heterocyclic base present in DNA and is thermally stable in aqueous solution. However, thymine is highly reactive in the presence of free radicals, such as OH, with which it reacts to form products which may be readily identified using HPLC techniques.

### Example 1

### Test to determine stability of thymine solutions when thermally heated.

Solutions of 1 x 10⁻³ mol/dm³ thymine in water were autoclaved under pressure at both 120 and 140°C. The solutions were then analysed using HPLC techniques and no breakdown products were detected.

### Example 2

### Detection of damaged electrode by use of inventive technique.

Ohmic heating was carried out in a small-scale static apparatus, comprising a heating cell consisting of a PVDF rectangular container (16cm x 7cm x 5cm). The container was filled with a 1 x 10⁻³ mol/dm³ aqueous thymine solution and placed in a cyclindrical, stainless steel chamber which was then sealed and filled with nitrogen at a pressure of 4 bar. An electric current of 6-8 amps (50 Hz) at 240 volts was then passed between platinum/iridium plated electrodes, immersed in the thymine solution. The rise in the temperature of the solution was monitored, using thermacouples placed at various positions therein, and heating was stopped when the solution reached 140°C, after which the solution was allowed to cool down to room temperature. On average, 60-90 seconds was required to reach this temperature. The solution was then analysed using HPLC techniques and breakdown products were detected.

The electrodes had visibly pitted and scratched surfaces, to the extent that the underlying titanium metal was exposed and it is considered that free radical formation took place on these electrodes, either due to bi-metallic effects or localised regions of high current density on the electrode surfaces. It is possible that other factors contributed to breakdown products being observed. Firstly, the electrode/solution contact time in this example was far greater than in the plant shown in Figure 1 (when operated as hereinbefore described) and the electrode area to solution volume was also far greater in this example, than it is in the plant illustrated in Figure 1.

The present invention, thus, provides a route allowing on-going monitoring of an ohmic heating process, particularly in order to provide an early detection of wear or damage to the electrodes. The thymine or other test substance, such as linoleic acid, can be incorporated into the sodium sulphate solution, which is used, on a daily basis, to start up the ohmic heating plant shown in Figure 1 for bringing it to equilibrium prior to the introduction of the flowable medium (food products). The frequency of the measurements, daily, weekly or monthly, will be determined by the degree of risk related to the products being processed.

## Claims

1. A method of testing a procedure or apparatus for processing a flowable material, characterised by carrying out the procedure or operating the apparatus on a test fluid comprising a test substance which remains substantially unmodified under the intended influence of the apparatus or procedure and which, in the presence of a free radical, is modified into a detectably different form, and thereafter, analysing the test fluid for said detectably different form.

2. A test method as claimed in claim 1, wherein the apparatus comprises an electrode (10) and the method comprises contacting the electrode (10) with the test fluid, feeding an alternating electric current to the electrode (10) and, thereafter,analysing the test fluid for said detectably different form.

3. A test method as claimed in claim 2, wherein the test fluid is electrically conducting and electric current is passed through the test fluid, to and from the electrode (10).

4. A test method as claimed in any of the preceding claims, wherein the apparatus comprises an electrolytic cell and the method comprises allowing the test fluid to enter the cell, passing an alternating electric current through the fluid and, thereafter, analysing the test fluid for said detectably different form.

5. A test method as claimed in any of the preceding claims, wherein the flowable material is electrically conducting.

6. A method as claimed in any of the preceding claims, wherein the procedure or operating the apparatus heats the test fluid to an elevated temperature at which substantially all of the heated test substance will not be modified into said detectably different form by the heat alone.

7. A test method as claimed in claim 6, wherein the procedure is an ohmic heating process, and the method involves passing an alternating electric current through the test fluid, in order to raise its temperature to said elevated temperature.

8. A test method as claimed in any of claims 2 to 4, wherein at least two electrodes (10) are contacted with the fluid and the alternating current is passed between the electrodes (10).

9. A test method as claimed in any of the preceding claims, wherein the test fluid and substance are substantially stable, to the effect of heat alone, up to 120° C and, preferably, up to 140°C.

10. A test method as claimed in any of the preceding claims, wherein the test substance is a heterocyclic base, amino acid or an essential fatty acid.

11. A test method as claimed in claim 10, wherein the test substance is a pyrimidine or a purine.

12. A test method as claimed in claim 11, wherein the test substance is thymine.

13. A test method as claimed in claim 10, wherein the test substance is the essential fatty acid linoleic acid.

14. A test method as claimed in any of claims 10 to 13, wherein the test fluid is an aqueous solution and, preferably, includes sodium sulphate.

15. A method of operating an ohmic heating plant, including the step of carrying out a test method as claimed in any of the preceding claims.

16. A method of operating an ohmic heating plant, as claimed in claim 15, wherein the test fluid is passed through an ohmic heating column (3), while an alternating electric current is passed through electrodes (10) within said column (3) and the fluid is analysed for the detectably different form after having passed through the column (3).

17. A test method as claimed in any of claims 1 to 13, wherein the fluid is analysed using HPLC techniques.

## Patentansprüche

1. Verfahren zur Untersuchung eines Verfahrens oder einer Vorrichtung zur Verarbeitung eines strömungsfähigen Materials, dadurch gekennzeichnet, daß das Verfahren oder der Betrieb der Vorrichtung mit einer Testflüssigkeit durchgeführt wird, welche eine Testsubstanz enthält, die bei der angestrebten Einwirkung der Vorrichtung oder des Verfahrens im wesentlichen unverändert bleibt und die in Anwesenheit eines freien Radikals in eine nachweisbare, unterschiedliche Form umgewandelt wird, und daß die Testflüssigkeit anschließend auf die nachweisbare, unterschiedliche Form untersucht wird.

2. Testverfahren nach Anspruch 1, bei dem die Vorrichtung eine Elektrode (10) enthält und bei dem das Verfahren Inkontakt bringen der Elektrode (10) mit der Testflüssigkeit, Anlegen eines elektrischen Wechselstroms an die Elektrode (10) und anschließend Untersuchen der Testflüssigkeit auf die nachweisbare, unterschiedliche Form umfaßt.

3. Testverfahren nach Anspruch 2, bei dem die Testflüssigkeit elektrisch leitend ist und bei dem durch die Testflüssigkeit zu und von der Elektrode (10) elektrischer Strom geleitet wird.

4. Testverfahren nach einem der vorhergehenden Ansprüche, bei dem die Vorrichtung eine elektrolytische Zelle enthält und bei dem das Verfahren Einbringen der Testflüssigkeit in die Zelle, Leiten eines elektrischen Wechselstroms durch die Flüssigkeit und anschließend Untersuchen der Testflüssigkeit auf die nachweisbare, unterschiedliche Form umfaßt.

5. Testverfahren nach einem der vorhergehenden Ansprüche, bei dem das strömungsfähige Material elektrisch leitend ist.

6. Verfahen nach einem der vorhergehenden Ansprüche, bei dem das Verfahren oder der Betrieb der Vorrichtung die Testflüssigkeit auf eine erhöhte Temperatur erwärmt, bei der die erhitzte Testsubstanz im wesentlichen nicht vollständig durch die Wärme allein in die nachweisbare, unterschiedliche Form verändert wird.

7. Testverfahren nach Anspruch 6, bei dem das Verfahren ein Widerstands-Erwärmungsverfahren ist und bei dem das Verfahren Leiten eines elektrischen Wechselstroms durch die Testflüssigkeit umfaßt, um deren Temperatur auf die erhöhte Temperatur zu bringen.

8. Testverfahren nach einem der Ansprüche 2 bis 4 bei dem mindestens zwei Elektroden (10) mit der Flüssigkeit in Kontakt treten und bei dem der Wechselstrom zwischen den Elektroden (10) geleitet wird.

9. Testverfahren nach einem der vorhergehenden Ansprüche, bei dem die Testflüssigkeit und die Substanz hinsichtlich allein der Wärme bis zu 120 °C und vorzugsweise bis zu 140 °C im wesentlichen stabil sind.

10. Testverfahren nach einem der vorhergehenden Ansprüche, bei dem die Testsubstanz eine heterozyklische Base, eine Aminosäure oder eine essentielle Fettsäure ist.

11. Testverfahren nach Anspruch 10, bei dem die Testsubstanz ein Pyrimidin oder ein Purin ist.

12. Testverfahren nach Anspruch 11, bei dem die Testsubstanz Thymin ist.

13. Testverfahren nach Anspruch 10, bei dem die Testsubstanz die essentielle Fettsäure Linolsäure ist.

14. Testverfahren nach einem der Ansprüche 10 bis 13, bei dem die Testflüssigkeit eine wässrige Lösung ist und vorzugsweise Natriumsulfat enthält.

15. Verfahren zum Betrieb einer Widerstands-Erwärmungsanlage, welche den Schritt der Durchführung eines Testverfahrens nach einem der vorhergehenden Ansprüche enthält.

16. Verfahren zum Betrieb einer Widerstands-Erwärmungsanlage nach Anspruch 15, bei dem die Testflüssigkeit durch eine Widerstands-Erwärmungssäule (3) geleitet wird, während durch die Elektroden (10) in der Säule (3) ein elektrischer Wechselstrom geleitet wird und bei dem die Flüssigkeit nach Durchleiten durch die Säule (3) auf die nachweisbare, unterschiedliche Form untersucht wird.

17. Testverfahren nach einem der Ansprüche 1 bis 13, bei dem die Flüssigkeit unter Verwendung von HPLC-Techniken untersucht wird.

## Revendications

1. Méthode pour tester un mode opératoire ou un appareil pour le traitement d'une matière apte à l'écoulement, caractérisée par la mise en oeuvre du mode opératoire ou le fonctionnement de l'appareil sur un fluide d'essai comprenant une substance d'essai qui reste pratiquement non modifiée sous l'influence prévue de l'appareil ou du mode opératoire et qui, en présence d'un radical libre, est modifiée en une forme différente de manière détectable, puis l'analyse du fluide d'essai pour déterminer ladite forme différente de manière détectable.

2. Méthode d'essai suivant la revendication 1, dans laquelle l'appareil comprend une électrode (10), la méthode comprenant la mise en contact de l'électrode (10) avec le fluide d'essai, l'envoi d'un courant électrique alternatif à l'électrode (10), puis l'analyse du fluide d'essai pour déterminer la forme différente de manière détectable.

3. Méthode d'essai suivant la revendication 2, dans laquelle le fluide d'essai est électriquement conducteur et un courant électrique est passé à travers le fluide d'essai, à l'électrode (10) et hors de cette électrode.

4. Méthode d'essai suivant l'une quelconque des revendications précédentes, dans laquelle l'appareil comprend une cellule électrolytique, ladite méthode comprenant les étapes consistant à laisser le fluide d'essai pénétrer dans la cellule, à faire passer un courant électrique alternatif à travers le fluide, puis à analyser le fluide d'essai pour déterminer ladite forme différente de manière détectable.

5. Méthode d'essai suivant l'une quelconque des revendications précédentes, dans laquelle la matière apte à l'écoulement est électriquement conductrice.

6. Méthode suivant l'une quelconque des revendications précédentes, dans laquelle le mode opératoire ou le fonctionnement de l'appareil provoque l'échauffement du fluide d'essai à une température élevée à laquelle pratiquement la totalité de la substance d'essai chauffée n'est pas modifiée en ladite forme différente de manière détectable par la chaleur seule.

7. Méthode d'essai suivant la revendication 6, dans laquelle le mode opératoire est un procédé de chauffage ohmique, ladite méthode comprenant le passage d'un courant électrique alternatif à travers le fluide d'essai, afin de porter sa température à ladite température élevée.

8. Méthode d'essai suivant l'une quelconque des revendications 2 à 4, dans laquelle au moins deux électrodes (10) sont mises en contact avec le fluide et le courant alternatif est passé entre les électrodes (10).

9. Méthode d'essai suivant l'une quelconque des revendications précédentes, dans laquelle le fluide d'essai et la substance d'essai sont essentiellement stables vis-à-vis de l'effet de la chaleur seule, jusqu'à 120°C et de préférence jusqu'à 140°C.

10. Méthode d'essai suivant l'une quelconque des revendications précédentes, dans laquelle la substance d'essai est une base hétérocyclique, un aminoacide ou un acide gras essentiel.

11. Méthode d'essai suivant la revendication 10, dans laquelle la substance d'essai est une pyrimidine ou une purine.

12. Méthode d'essai suivant la revendication 11, dans laquelle la substance d'essai est la thymine.

13. Méthode d'essai suivant la revendication 10, dans laquelle la substance d'essai est un acide gras essentiel consistant en acide linoléique.

14. Méthode d'essai suivant l'une quelconque des revendications 10 à 13, dans laquelle le fluide d'essai est une solution aqueuse et comprend de préférence le sulfate de sodium.

15. Procédé pour faire fonctionner une installation de chauffage ohmique, comprenant l'étape de mise en oeuvre d'une méthode d'essai suivant l'une quelconque des revendications précédentes.

16. Procédé pour faire fonctionner une installation de chauffage ohmique, suivant la revendication 15, dans lequel le fluide d'essai est passé à travers une colonne de chauffage ohmique (3), tandis qu'un courant électrique alternatif est passé à travers les électrodes (10) à l'intérieur de ladite colonne (3) et le fluide est analysé pour déterminer la forme différente de manière détectable après son passage à travers la colonne (3).

17. Méthode d'essai suivant l'une quelconque des revendications 1 à 13, dans laquelle le fluide est analysé par des techniques de chromatographie en phase liquide à hautes performances (CLHP).
